# DEMANDE DE BREVET EUROPEEN

(11) **EP 0 928 603 A1**
(43) Date de publication de la demande: **14.07.1999**
(21) Numéro de dépôt: 99400052.9
(22) Date de dépôt: 11.01.1999
(51) Int. Cl.: A61B 17/70

(54) **Dispositif de liaison intervertébrale**

(30) Priorité: 12.01.1998 FR 9800212
(71) Demandeur: Sessa, Salvatore, 92300 Levallois Perret (FR)
(72) Inventeur: Sessa, Salvatore, 92300 Levallois Perret (FR)

(57) **Abrégé**

La présente invention concerne un dispositif de liaison intervertébrale du type constitué, d'une part, par des plaques (2) ou tiges rigides (2A) disposées longitudinalement de part et d'autre desdites vertèbres et reliées transversalement entre elles par une barre d'union et, d'autre part, par au moins un ligament textile artificiel souple reliant ladite barre (6) à une vertèbre libre caractérisé en ce que la barre d'union (6) comporte deux éléments de fixation (8 et 9) destinés à sa liaison avec les extrémités libres (7Aa et 7Ba) de deux demi-bras ligamentaires (7A ou 7B) constituant un ligament souple (7) ayant préalablement entouré librement une apophyse épineuse (10) sus-jacente non arthrodésée.

## Description

La présente invention concerne un dispositif de liaison intervertébrale du type constitué, d'une part, par des plaques ou tiges rigides disposées longitudinalement de part et d'autre desdites vertèbres par l'intermédiaire de vis pédiculaires ou crochets et de moyens de fixation les surmontant, lesdites plaques ou tiges étant reliées transversalement entre elles par une barre d'union dont les extrémités coopèrent avec lesdits moyens de fixation pour assurer une arthrodèse et, d'autre part, par au moins un ligament textile artificiel souple reliant ladite barre d'union à une vertèbre libre non arthrodésée située à une certaine distance de ladite barre d'union.

Un tel dispositif a pour rôle, en associant une barre d'union rigide et une liaison ligamentaire interépineuse, de réaliser une ligamentoplastie intervertébrale de la zone de jonction rachidienne entre une partie rigide arthrodésée et une partie libre.

Un tel dispositif a pour objectif de :
- faciliter l'ostéo-intégration de la greffe osseuse grâce aux micro-mouvements résiduels,
- prévenir une rupture brutale des contraintes au niveau des segments adjacents, à l'origine de déstabilisation iatrogène ascendante.

En effet, si les résultats immédiats de la chirurgie lombaire sont satisfaisants dans les 70 à 85 % des cas selon les différentes techniques, les résultats secondaires sont souvent moins bons, à cause de l'apparition au-dessus de l'arthrodèse d'une instabilité ou d'une sténose (jusqu'à 40 % selon les différentes séries). L'origine de ces problèmes est sûrement la rupture dans l'absorption des contraintes.

Pour obtenir ces résultats, il est connu un système semi-rigide ligamentaire, associant une ostéosynthèse rigide et une ligamentoplastie interépineuse à la zone de jonction, afin d'obtenir un certain degré d'élasticité contrôlée, en bout de fixation. Le but est d'éviter le phénomène de la néo-charnière, sus-jacent l'arthrodèse, responsable de l'arthrose des articulations interapophysaires et de la dégénérescence discale, facteurs de mauvais résultats secondaires.

La ligamentoplastie est un système semi-contraint connu qui évite les contacts interfacettaires et interépineux appuyés, tandis que l'axe de rotation vertébral est stabilisé en arrière de l'espace postérieur interdiscal. La remise en lordose permet une précontrainte postérieure permettant le verrouillage lombaire automatique.

Parmi les techniques utilisées à ce jour, les plus fréquentes sont les ligamentoplasties interpédiculaires avec fixation sur les têtes des vis vertébrales et les ligamentoplasties interépineuses avec l'utilisation de cale métallique ou en polyéthylène, de coussinet interépineux, de prothèse intervertébrale en titane, d'anneau interépineux en Dacron ou cage autobloquante.

Les ligamentoplasties interpédiculaires sont critiquables car il s'agit d'une technique agressive et iatrogène. En effet, l'utilisation de vis pédiculaires qui ont en général un gros diamètre, est en elle-même un geste iatrogène à cause du risque radiculaire lié à la visée pédiculaire et rend difficile une éventuelle reprise chirurgicale. Le site d'implantation des vis contraint à sacrifier partiellement les massifs articulaires susjacents (d'une vertèbre a priori saine) et entame la cohésion facettaire capsulo-ligamentaire, avec le résultat d'un facet-syndrome douloureux. Par ailleurs, les ligaments étant au contact des zones interarticulaires, ils ont tendance à travailler en hyperappui, ce qui aggrave l'hyperpression des facettes. Le relâchement capsulo-ligamentaire, associé à une dégénérescence discale, est à l'origine d'épisodes lombalgiques aigus qui témoignent d'une instabilité lombaire évolutive.

Enfin, les ligamentoplasties interpédiculaires déplacent en avant et latéralement l'action de la ligamentoplastie et diminuent la mobilité du segment concerné seulement en flexion frontale, en contrôlant de façon très limitée l'extension et surtout les translations latérales. En revanche, elles ne coupent pas les ponts pour une chirurgie d'arthrodèse et, d'autre part, il est possible de réaliser un système semi-rigide par une fixation mixte barre d'union vertébrale et ligament, dite « B.U.L. », toujours avec vis pédiculaires, de chaque côté des lames vertébrales. Un dispositif de ce type est décrit dans la demande de brevet français N° 2.749.155.

Les ligamentoplasties interépineuses également connues sont plus efficaces du point de vue biomécanique car elles permettent d'obtenir un meilleur moment stabilisateur. En effet, le centre permanent de rotation des vertèbres étant situé dans le tiers postérieur du disque, les apophyses épineuses sont anatomiquement beaucoup plus éloignées du tiers postérieur du disque que la partie postérieure des pédicules. Placées à distance des centres de rotation, elles sont actives par le bras de levier des apophyses épineuses (action du ligament artificiel). Par conséquent, la ligamentoplastie interépineuse du segment libre sus-jacent l'arthrodèse permet de limiter la mobilité (15-20 %), sans la supprimer, en contrôlant les mouvements en flexion-extension et en inclinaison latérale et les translations sagittales par une mise en tension contrôlée. La mise en place possible, mais pas nécessaire, d'un coussinet interépineux qui agit comme un amortisseur, évite le contact interépineux en extension et, par effet délordosant, la surcharge en compression des articulations postérieures, tout en réouvrant les foramens.

Avec les dispositifs actuels, les ligamentoplasties interépineuses, sans ancrage pédiculaire, posent aussi des problèmes de fiabilité technique car la force de mise en tension est relativement modérée et difficile à contrôler et la fixation du ligament à lui-même est très aléatoire et mécaniquement peu résistante.

La présente invention a pour but de remédier à l'ensemble de ces inconvénients et concerne à cet effet un dispositif de liaison intervertébrale du type constitué, d'une part, par des plaques ou tiges rigides disposées longitudinalement de part et d'autre desdites vertèbres par l'intermédiaire de vis pédiculaires ou crochets et des moyens de fixation les surmontant, lesdites plaques ou tiges étant reliées transversalement entre elles par une barre d'union dont les extrémités coopèrent avec lesdits moyens de fixation pour assurer une arthrodèse et, d'autre part, par au moins un ligament textile artificiel souple reliant ladite barre à une vertèbre libre non arthrodésée située à une certaine distance de ladite barre d'union ligamentaire B.U.L., caractérisé en ce que la barre d'union (B.U.L.) comporte deux éléments de fixation destinés à sa liaison avec les extrémités libres de deux demi-bras ligamentaires constituant un ligament souple ayant préalablement entouré librement une apophyse épineuse sus-jacente non arthrodésée, de manière à ce que chaque demi-bras ligamentaire exerce un effet de rappel vers la ligne médiane des vertèbres, assurant l'autocontrôle de leur alignement lors d'une mise en tension.

Ainsi et contrairement aux dispositifs connus précités ci-dessus, la mise en tension du ligament interépineux, fiablement fixé de chaque côté sur la barre d'union transversale ligamentaire (B.U.L.), favorise la transmission progressive des contraintes entre le segment rigide et le segment mobile.

De cette façon, une mise en tension contrôlée peut être assurée et cela est le meilleur garant de l'efficience des principes biomécaniques.

En fait, la différence fondamentale de l'invention par rapport aux dispositifs de l'art antérieur réside dans le fait que la mise en oeuvre du ligament souple ne nécessite pas la mise en place de vis pédiculaires d'aucune sorte.

Ainsi, l'invention s'adapte sur toutes les instrumentations d'arthrodèse vertébrale, et permet de remédier aux divers inconvénients des ligamentoplasties interépineuses et interpédiculaires, ne disposant pas de dispositif objet de l'invention. Ce dernier permet de pouvoir utiliser la fixation ligamentaire de façon systématique, avec un geste chirurgical simple en fin d'intervention, nécessitant seulement 5 à 10 mn pour sa mise en place.

La présente invention concerne également les caractéristiques qui ressortiront au cours de la description qui va suivre et qui devront être considérées isolément ou selon toutes leurs combinaisons techniques possibles.

Cette description, donnée à titre d'exemple non limitatif, fera mieux comprendre comment l'invention peut être réalisée, en référence aux dessins annexés sur lesquels :
- la figure 1 représente en perspective un dispositif de liaison intervertébrale selon l'invention mettant en oeuvre des plaques fixées sur des têtes de vis pédiculaires ;
- la figure 2 représente en perspective un dispositif de liaison intervertébrale selon l'invention mettant en oeuvre des tiges cylindriques associées à des crochets.

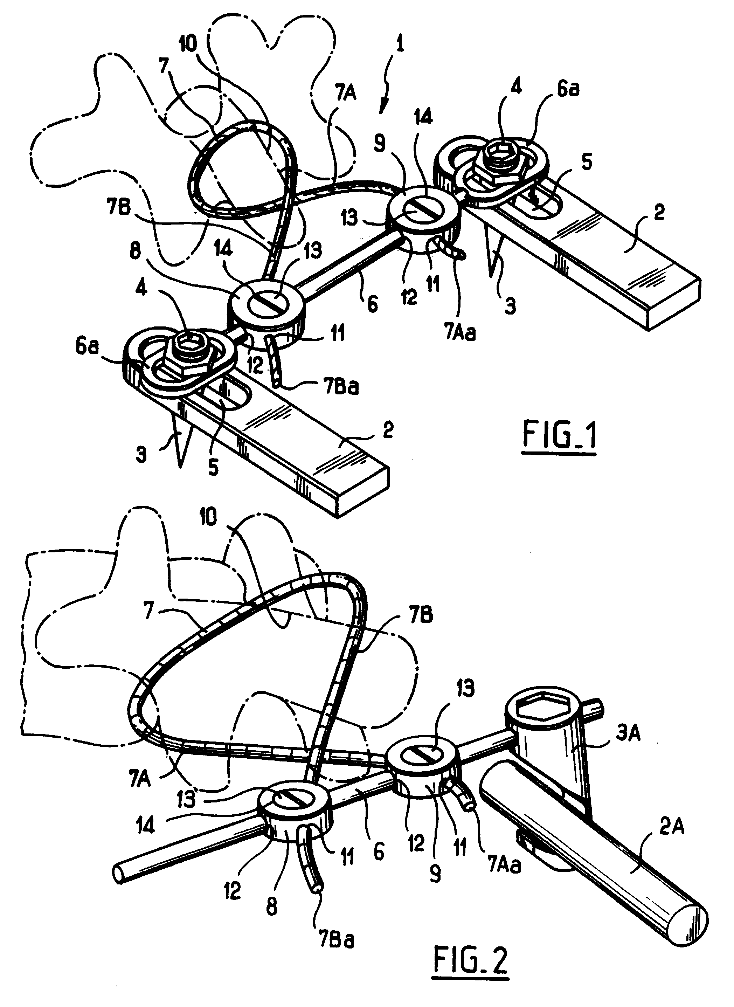

Le dispositif de liaison 1 désigné dans son ensemble sur la figure 1, a pour but d'immobiliser deux ou plusieurs segments vertébraux (non représentés).

Il est constitué de manière connue par des plaques 2 disposées longitudinalement de part et d'autre desdites vertèbres pour assurer une arthrodèse.

Ceci est effectué par l'intermédiaire de vis pédiculaires 3 et de moyens de fixation 4 surmontant lesdites vis 3, et qui sont généralement constitués par une extrémité filetée de celles-ci et d'un écrou adapté.

Lesdites plaques disposent généralement de lumières 5 pour permettre leur réglage en position souhaitée.

Le dispositif comprend également de manière connue une barre d'union 6 reliant transversalement entre elles les plaques 2, les extrémités 6a de ladite barre d'union 6 coopérant avec les moyens de fixation 4 des plaques 2.

L'exemple de réalisation selon la figure 2 diffère essentiellement du précédent en ce que la plaque 2 est remplacée par une tige cylindrique rigide 2A et en ce que celle-ci coopère non pas avec une vis pédiculaire 3, mais avec un crochet 3A.

Selon l'invention se rapportant tant à l'exemple de la figure 1 qu'à celui de la figure 2, la barre d'union 6 comporte deux éléments de fixation 8 et 9 destinés à sa liaison avec les extrémités libres 7Aa et 7Ba de deux demi-bras ligamentaires 7A ou 7B constituant un ligament souple 7 ayant préalablement entouré librement une apophyse épineuse 10 sus-jacente non arthrodésée, de manière à ce que chaque demi-bras ligamentaire 7A, 7B exerce un effet de rappel vers la ligne médiane des vertèbres, assurant l'autocontrôle de leur alignement lors d'une mise en tension.

Selon les présents exemples de réalisation des figures 1 et 2, le ligament souple 7 entoure l'apophyse épineuse 10 d'une vertèbre non arthrodésée en formant une boucle ouverte, avant d'être reliée par ses extrémités libres 7Aa, 7Ba aux deux éléments de fixation 8 et 9 disposés sur la barre d'union ligamentaire (B.U.L.) 6.

Bien entendu, le ligament souple 7 pourrait également entourer l'apophyse épineuse 10 d'une vertèbre non arthrodésée, ses deux demi-bras 7A, 7B le formant, se croisant avant d'être reliés par ses extrémités libres 7Aa, 7Ba aux deux éléments de fixation 8 et 9 disposés sur la barre d'union 6.

Selon les présents modes de réalisation, les éléments de fixation 8 et 9 des extrémités 7Aa, 7Ba du ligament souple 7 réalisés sur la barre d'union 6 sont symétriques par rapport aux extrémités de celle-ci.

Mais bien entendu, les mêmes éléments des fixations 8 et 9 pourraient être disposés sur la barre d'union 6 de façon asymétrique.

De la même manière, les éléments de fixation 8 et 9 des extrémités 7Aa, 7Ba du ligament souple 7 sont réalisés de façon monobloc avec la barre d'union 6, ou encore rapportés de manière coulissante sur ladite barre d'union 6.

Selon le présent exemple de réalisation, nullement limitatif, chacun des éléments de fixation 8 et 9 des extrémités 7Aa, 7Ba du ligament souple 7 est constitué par un taquet qui est traversé de part en part par la barre d'union 6 par un trou 12 de celle-ci et qui est traversé également par un trou de passage 11 desdites extrémités du ligament 7 dans lequel débouche un trou fileté 14, perpendiculaire à son axe longitudinal, qui est destiné à recevoir une vis d'immobilisation 13 des extrémités 7Aa. 7Ba du ligament 7, après que celui-ci ait entouré une apophyse épineuse 10 d'une vertèbre non arthrodésée.

Selon une autre caractéristique de l'invention, la vis d'immobilisation 14 des extrémités 7Aa, 7Ba du ligament 7 bloque simultanément les taquets 8 et 9 sur la barre d'union (B.U.L.) 6.

Après la fixation de la barre d'union 6 sur l'instrumentation vertébrale, en général un niveau plus bas de l'étage de la ligamentoplastie interépineuse (par exemple niveau L5 pour ligamentoplastie L3-L4), à titre d'exemple mais non limitatif, un brin du ligament 7 est passé dans le trou 11 d'un des taquets 8 et fixé par serrage de la vis 13 qui, grâce à la surface large de contact qu'elle présente avec le ligament, ne risque pas de déchirer ce dernier. Cette vis 13 peut présenter, à son extrémité ligamentaire, une surface arrondie intégrée dans le corps de la vis 13 qui ne tourne pas lors du vissage, mais bloque seulement le ligament 7. Ensuite, le ligament 7 est passé en boucle ou en croix autour des épineuses sus-jacentes 10 (deux ou plusieurs), croisé sous le bord inférieur de l'épineuse sus-jacente et, enfin, passé dans le trou 11 de l'autre taquet 9. Le ligament 7 est donc mis en tension à l'aide d'une pince adaptée et fixé par serrage de la deuxième vis 13.

Dans le cas où la barre d'union transversale 6 est de section orthogonale sous forme d'une plaque (figure 1) ou sous forme d'une tige de section circulaire (figure 2), les taquets 8 et 9 peuvent être monoblocs par rapport à la barre de liaison 6 ou rapportés symétriquement ou asymétriquement par rapport à ses extrémités.

Dans ce système de fixation ligamentaire des épineuses adjacentes, l'arthrodèse par une barre d'union transversale 6 équipée de deux taquets 8, 9 est préférable à une simple barre transversale qui serait sans taquet de fixation ligamentaire, mais tous les moyens de fixation du ligament interépineux 7 sur la barre transversale (B.U.L.) 6 peuvent être valables et correspondent au principe du dispositif objet de l'invention.

Selon une autre caractéristique de l'invention, afin d'éviter la détérioration de la partie de ligament situé au droit de la vis 13 dans le taquet 8 lors du serrage de ladite vis 13, celle-ci est pourvue à son extrémité d'un élément de contact intermédiaire, monté libre en rotation sur ladite extrémité, de manière à éviter toute friction sur le ligament.

## Revendications

1. Dispositif de liaison intervertébrale du type constitué, d'une part, par des plaques (2) ou tiges rigides (2A) disposées longitudinalement de part et d'autre desdites vertèbres par l'intermédiaire de vis pédiculaires (3) ou crochets (3A) et des moyens de fixation (4) les surmontant, lesdites plaques (2) ou tiges (2A) étant reliées transversalement entre elles par une barre d'union dont les extrémités (6a) coopèrent avec lesdits moyens de fixation (4) pour assurer une arthrodèse et, d'autre part, par au moins un ligament textile artificiel souple reliant ladite barre (6) à une vertèbre libre non arthrodésée située à une certaine distance de ladite barre d'union ligamentaire (B.U.L.) (6), caractérisé en ce que la barre d'union (B.U.L.) (6) comporte deux éléments de fixation (8 et 9) destinés à sa liaison avec les extrémités libres (7Aa et 7Ba) de deux demi-bras ligamentaires (7A ou 7B) constituant un ligament souple (7) ayant préalablement entouré librement une apophyse épineuse (10) sus-jacente non arthrodésée, de manière à ce que chaque demi-bras ligamentaire (7A, 7B) exerce un effet de rappel vers la ligne médiane des vertèbres, assurant l'autocontrôle de leur alignement lors d'une mise en tension.

2. Dispositif de liaison selon la revendication 1, caractérisé en ce que les éléments de fixation (8 et 9) des extrémités (7Aa, 7Ba) du ligament souple (7) réalisés sur la barre d'union (6) sont symétriques par rapport aux extrémités de celle-ci.

3. Dispositif de liaison selon la revendication 1, caractérisé en ce que les éléments de fixation (8 et 9) des extrémités (7Aa, 7Ba) du ligament souple (7) réalisés sur la barre d'union (6) sont asymétriques par rapport aux extrémités de celle-ci.

4. Dispositif de liaison selon la revendication 2 ou 3, caractérisé en ce que les éléments de fixation (8 et 9) des extrémités (7Aa, 7Ba) du ligament souple (7) sont réalisés de façon monobloc avec la barre d'union (6).

5. Dispositif de liaison selon la revendication 2 ou 3, caractérisé en ce que les éléments de fixation (8 et 9) des extrémités (7Aa, 7Ba) du ligament souple (7) sont rapportés de manière coulissante sur la barre d'union (6).

6. Dispositif de liaison selon l'une des revendications 1 à 5, caractérisé en ce que chacun des éléments de fixation (8 et 9) des extrémités (7Aa, 7Ba) du ligament souple (7) est constitué par un taquet qui est traversé de part en part par la barre d'union (6) par un trou (12) de celle-ci et qui est traversé également par un trou de passage (11) desdites extrémités du ligament (7) dans lequel débouche un trou fileté (14), perpendiculaire à son axe longitudinal, qui est destiné à recevoir une vis d'immobilisation (13) des extrémités (7Aa, 7Ba) du ligament (7), après que celui-ci ait entouré une apophyse épineuse (10) d'une vertèbre non arthrodésée.

7. Dispositif de liaison selon la revendication 6, caractérisé en ce que la vis d'immobilisation (14) des extrémités (7Aa, 7Ba) du ligament (7) bloque simultanément les taquets (8 et 9) sur la barre d'union (6).
